# EUROPEAN PATENT SPECIFICATION

(11) **EP 4 724 434 B1**
(45) Date of publication and mention of the grant of the patent: **02.12.2026**
(21) Application number: 24794005.9
(22) Date of filing: 15.10.2024
(51) Int. Cl.: C07D 317/70, A61K 8/49, A61Q 13/00

(54) **AMBERGRIS ISOMERS AND MIXTURES THEREOF**
AMBRA-ISOMERE UND DEREN MISCHUNGEN
ISOMÈRES DE L'AMBRE GRIS ET LEURS MÉLANGES

(43) Date of publication of application: 15.04.2026
(73) Proprietor: Symrise AG, 37603 Holzminden Niedersachsen (DE)
(72) Inventor: LAMBRECHT, Stefan, 37619 Hehlen (DE); EH, Marcus, 37603 Holzminden (DE); ONGOUTA, Jekaterina, 37627 Stadtoldendorf (LV); HILMER, Silke, 37603 Holzminden (DE)
(74) Representative: Eisenführ Speiser
(86) International application number: PCT/EP2024/078969
(87) International publication number: WO 2025/247515

(56) References cited:
- EP-A1- 2 287 158
- WO-A1-2019/141881

## Description

The present invention primarily relates to a compound of formula (epi-I) or a compound of formula (epi-II) as defined herein. Moreover, the present invention relates to mixtures comprising the compound of formula (epi-I) and/or the compound of formula (epi-II) as defined herein, and to fragrance compositions and perfumed products as defined herein. The present invention further relates to a method for manufacturing a perfumed product as defined herein, to a method for providing and/or enhancing one or more odour note(s) selected from the group consisting of ambergris, white amber and woody to/in a product as defined herein, and to a method (a) for enhancing the or one or several pleasant odour impression(s) of one or more pleasantly smelling substance(s), and/or (b) for masking or reducing the or one or several unpleasant odour impression(s) of one or more unpleasantly smelling substance(s) as defined herein. Moreover, the present invention relates to the use of a compound or mixture or fragrance composition as defined herein as a fragrance or to (a) mask or reduce the or one or several unpleasant odour impression(s) of one or more unpleasantly smelling substance(s) and/or (b) enhance the or one or several pleasant odour impression(s) of one or more pleasantly smelling substance(s). Lastly, the present invention relates to the use of a perfumed product as defined herein to perfume textiles, hair, skin, surfaces and/or ambient air.

Further aspects of the present invention will arise from the description below, in particular from the examples, as well as from the attached patent claims.

Ambrostar^{®} is an interesting perfume ingredient that is characterized by a strong odour of wood and ambergris with notes of white amber. Ambrostar^{®} has been described as containing one or both of the diastereomers of formulae (I) and (II) 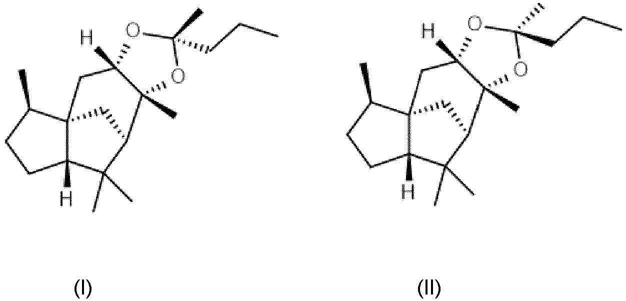 (cf. e.g. EP2287158 A1). The compound of formula (I) has the CAS number 1267350-23-1 and the compound of formula (II) has the CAS number 1267350-25-3.

However, the compounds of formulae (I) and (II) have a woody, ambra-like sensory profile with a light white amber note. It was thus an object of the present invention to provide improved compounds or mixtures with more intense white amber odour profiles. White amber means, in perfumery terms, the odour of aged natural ambergris, which represents a very valuable odour note. Further objects underlying the present invention follow from the description below and the patent claims.

According to a first aspect of the present invention, the stated object is achieved by a compound of formula (epi-I) 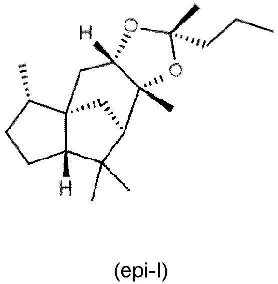 ((1R,3S,5S,7R,8R,10S,13S)-5,7,9,9,13-pentamethyl-5-propyl-4,6-dioxatetracyclo[6.5.1.01,10.03,7]tetradecane)
or a compound of formula (epi-II) 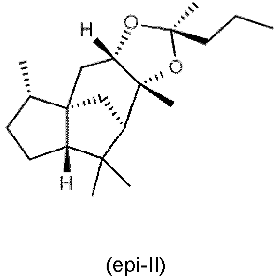 ((1R,3S,5R,7R,8R,10S,13S)-5,7,9,9,13-pentamethyl-5-propyl-4,6-dioxatetracyclo[6.5.1.01,10.03,7]tetradecane).

Surprisingly, it was found during the studies underlying the present invention that the presence of the two new isomers of Ambrostar^{®}, namely the compound of formula (epi-I) and the compound of formula (epi-II), leads to a particularly preferred isomer mixture. Moreover, it was found that they positively influence the sensorial properties of the mixture already in very small quantities.

Another aspect of the present invention relates to a mixture comprising or consisting of the compound of formula (epi-I) as defined herein and/or, preferably and, the compound of formula (epi-II) as defined herein and optionally at least one compound, preferably both compounds, selected from the group consisting of the compounds of formula (I) and (II) 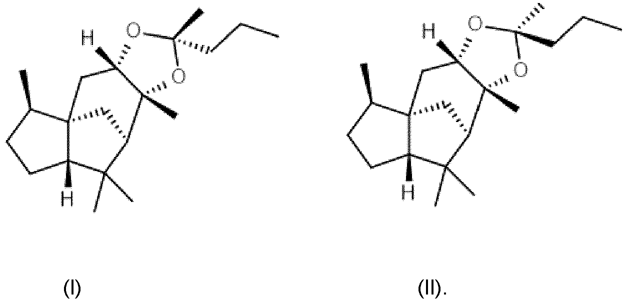 According to a preferred embodiment, the mixture comprises or consists of the compound of formula (epi-I) as defined herein and optionally at least one compound, preferably both compounds, selected from the group consisting of the compounds of formula (I) and (II) as defined herein.

According to another preferred embodiment, the mixture comprises or consists of the compound of formula (epi-II) as defined herein and optionally at least one compound, preferably both compounds, selected from the group consisting of the compounds of formula (I) and (II) as defined herein.

According to another preferred embodiment, the mixture comprises or consists of the compound of formula (epi-I) as defined herein and the compound of formula (epi-II) as defined herein and optionally the compound of formula (I) as defined herein.

According to another preferred embodiment, the mixture comprises or consists of the compound of formula (epi-I) as defined herein and the compound of formula (epi-II) as defined herein and optionally the compound of formula (II) as defined herein.

According to a most preferred embodiment, the mixture comprises or consists of the compound formula (epi-I) as defined herein, and the compound of formula (epi-II) as defined herein, and the compound of formula (I) as defined herein, and the compound of formula (II) as defined herein.

In another preferred embodiment of the mixture, the weight ratio of the total amount of the compound of formula (epi-I) as defined herein (if present) and of the compound of (epi-II) as defined herein (if present) to the total amount of the compound of formula (I) as defined herein (if present) and of the compound of formula (II) as defined herein (if present) is from about 0.01:99.99 to about 5:95, preferably from about 0.05:99.95 to about 4:96, more preferably from about 0.10:99.90 to about 3:97.

Another aspect of the present invention relates to a fragrance composition, preferably a perfume oil, comprising or consisting of
(i) the compound of formula (epi-I) as defined herein and/or, preferably and, the compound of formula (epi-II) as defined herein
   or
(ii) a mixture as defined herein
and
(iii) one or more additional fragrance substance(s).

Examples for additional fragrance substances, which may be advantageously used in fragrance compositions according to the present invention, are e.g. found in S. Arctander, Perfume and Flavor Materials, Vol. I and II, Montclair, N. J. 1969, own publisher, or K. Bauer et al., Common Fragrance and Flavor Materials, 4th Edition, Wiley-VCH, Weinheim 2001.

Explicitly mentioned are: Extracts of natural raw materials such as essential oils, concretes, absolues, resins, resinoids, balms, tinctures such as e.g. ambra tincture; amyris oil; angelica seed oil; angelica root oil; anise oil; valerian oil; basil oil; tree moss absolue; bay oil; mugwort oil; benzoe resin; bergamot oil; beeswax absolue; birch tar oil; bitter almond oil; savory oil; bucco leaf oil; cabreuva oil; cade oil; calmus oil; campher oil; cananga oil; cardamomen oil; cascarilla oil; cassia oil; cassie absolue; castoreum-absolue; cedar leaf oil; cedar wood oil; cistus oil; citronella oil; citrus oil; copaiva balm; copaiva balm oil; coriander oil; costus root oil; cumin oil; cypress oil; davana oil; dill weed oil; dill seed oil; eau de brouts absolue; oak wood absolue; elemi oil; estragon oil; eucalyptus-citriodora-oil; eucalyptus oil; fennel oil; spruce needle oil; galbanum oil; galbanum resin; geranium oil; grapefruit oil; guajak wood oil; gurjun balm; gurjun balm oil; helichrysum absolue; helichrysum oil; ginger oil; iris root absolue; iris root oil; jasmin absolue; kalmus oil; chamomile oil blau; chamomile oil roman; carrot seed oil; kaskarilla oil; pine needle oil; spearmint oil; caraway oil; labdanum oil; labdanum absolue; labdanum resin; lavandin absolue; lavandin oil; lavender absolue; lavender oil; lemongrass oil; lovage oil; lime oil distilled; lime oil pressed; linaloe oil; litsea-cubeba oil; laurel leaf oil; macis oil; majoram oil; mandarin oil; massoi bark oil; mimosa absolue; musk grain oil; musk tincture; muscatel-sage-oil; nutmeg oil; myrrh absolue; myrrh oil; myrten oil; carnation leaf oil; carnation blowwom oil; neroli oil; olibanum absolue; olibanum oil; opopanax oil; orange blossom absolue; orange oil; origanum oil; palmarosa oil; patchouli oil; perilla oil; peru balm oil; parsil leaf oil; parsil sed oil; petitgrain oil; peppermint oil; pepper oil; piment oil; pine oil; poley oil; rose absolue; rosewood oil; rose oil; rosemary oil; sage oil dalmatinic; sage oil spanish; sandalwood oil; cellerie seed oil; spica lavender oil; star anise oil; styrax oil; marigold oil; fir needle oil; tea-tree oil; turpentine oil; thyme oil; tolu balm; tonka absolue; tuberose absolue; vanilla extract; violet leaf absolue; verbena oil; vetiver oil; juniper berry oil; wine yeast oil; vermouth oil; wintergreen oil; ylang oil; ysop oil; zibet absolue; cinnamon leaf oil; cinnamon bark oil as well as fractions thereof or, respectively, ingredients isolated therefrom;
single fragrances of the group of carbohydrates such as e.g. 3-carene; α-pinene; β-pinene; α-terpinene; γ-terpinene; p-cymol; bisabolene; camphene; caryophyllene; cedrene; farnesene; limonene; longifolene; myrcene; ocimene; valencene; (E,Z)-1,3,5-undecatriene; styrol; diphenylmethane;
of the group of aliphatic alcohols such as e.g. hexanol; octanol; 3-octanol; 2,6-dimethylheptanol; 2-methyl-2-heptanol; 2-methyl-2-octanol; (E)-2-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
of the group of aliphatic aldehydes and their acetals such as e.g. hexanal; heptanal; octanal; nonanal; decanal; undecanal; dodecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal;2,6,10-trimethyl-9-undecenal; 2,6,10-trimethyl-5,9-undecadienal; heptanaldiethylacetale; 1,1-dimethoxy-2,2,5-trimethyl-4-hexen; citronellyloxyacet aldehyde; 1-(1-methoxy-propoxy)-(E/Z)-3-hexene;
of the aliphatic ketones and their oximes such as e.g. 2-heptanone; 2-octanone; 3-octanone; 2-nonanone; 5-methyl-3-heptanone; 5-methyl-3-heptanonoxime; 2,4,4,7-tetramethyl-6-octen-3-one; 6-methyl-5-hepten-2-one;
of the aliphatic sulphur containing compounds such as e.g. 3-methylthio-hexanol; 3-methylthiohexyl acetate; 3-mercaptohexanol; 3-mercaptohexylacetat; 3-mercaptohexylbutyrat; 3-acetylthiohexyl acetate; 1-menthen-8-thiol;
of the aliphatic nitriles such as e.g. 2-nonenacid nitrile; 2-undecenacid nitrile; 2-tridecenacid nitrile; 3,12-tridecadienacid nitrile; 3,7-dimethyl-2,6-octadien-acid nitrile; 3,7-dimethyl-6-octenacid nitrile;
of the esters of aliphatic carboxylic acids such as e.g. (E)- and (Z)-3-hexenyl formiate; ethylaceto acetate; isoamyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl-isobutyrate; hexyl crotonate; ethyliso valerianate; ethyl-2-methylpentanoate; ethyl hexanoate; allyl hexanoate; ethyl heptanoate; allyl heptanoate; ethyl octanoate; ethyl-(E,Z)-2,4-decadienoate; methyl-2-octinate; methyl-2-noninate; allyl-2-isoamyloxy acetate; methyl-3,7-dimethyl-2,6-octadien-oat; 4-methyl-2-pentyl-crotonate;
of the acyclic terpene alcohols such as e.g. geraniol; nerol; lavadulol; nerolidol; farnesol; tetrahydrolinalool; tetrahydrogeraniol; 2,6-dimethyl-7-octen-2-ol; 2,6-dimethyloctan-2-ol; 2-methyl-6-methylen-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol 2,6-dimethyl-2,5,7-octatrien-1-ol; as well as their formiates, acetates, propionates, isobutyrates, butyrates, isovalerianates, pentanoates, hexanoates, crotonates, tiglinates and 3-methyl-2-butenoates;
of the acyclic terpene aldehydes and ketones such as e.g. citronellal; 7-methoxy-3,7-dimethyloctanal; 2,6,10-trimethyl-9-undecenal; geranyl acetone; as well as the dimethyl- and diethyl acetals of geranial, neral,
of the cyclic terpene alcohols such as e.g. menthol; isopulegol; alpha-terpineol; terpinenol-4; menthan-8-ol; menthan-1-ol; menthan-7-ol; borneol; isoborneol; linalooloxid; nopol; cedrol; ambrinol; vetiverol; guajol; as well as their formiates, acetates, propionates, isobutyrates, butyrates, isovalerianates, pentanoates, hexanoates, crotonates, tiglinates and 3-methyl-2-butenoates;
of the cyclic terpene aldehydes and ketones such as e.g. menthone; isomenthone; 8-mercaptomenthan-3-one; carvone; campher; fenchone; alpha-ionone; beta-ionone; alpha-n-methylionone; beta-n-methylionone; alpha-isomethylionone; beta-isomethyl-ionone; alpha-irone; beta-damascenone; 1-(2,4,4-trimethyl-2-cyclohexen-1-yl)-2-buten-1-one; 1,3,4,6,7,8a-hexahydro-1,1,5,5-tetramethyl-2h-2,4a-methano-naphthalen-8(5h)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; beta-sinensal; acetylated cedar wood oil (methylcedrylketon);
of the cyclic alcohols such as e.g. 4-tert.-butylcyclohexanol; 3,3,5-trimethylcyclohexanol; 3-isocamphylcyclohexanol; 2,6,9-trimethyl-z2,z5,e9-cyclododecatrien-1-ol; 2-isobutyl-4-methyltetrahydro-2h-pyran-4-ol;
of the cycloaliphatic alcohols such as e.g. alpha,3,3-trimethylcyclohexylmethanol;1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 3-methyl-5-(2,2,3-trimethyl-3-cyclopent-1-yl)-pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
of the cyclic and cycloaliphatic ethers such as e.g. cineol; cedrylmethylether; cyclododecylmethylether;1,1-dimethoxycyclododecan; (ethoxymethoxy)cyclo-dodecan; alpha-cedrenepoxid; 3a,6,6,9a-tetramethyldodecahydro-naphtho[2,1-b]furan; 3a-ethyl-6,6,9a-trimethyldodecahydronaphtho[2,1b]furan; 1,5,9-tri-methyl-13-oxabicyclo[10.1.0] trideca-4,8-dien; rosenoxide; 2-(2,4-dimethyl-3-cyclohexen-1-yl)-5-methyl-5-(1-methylpropyl)-1,3-dioxan;
of the cyclic and macrocyclic ketones such as e.g. 4-tert.-butylcyclohexanone; 2,2,5-trimethyl-5-pentylcyclopentanone; 2-heptylcyclopentanone; 2-pentyl-cyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopenta-decanone; 4-(1-ethoxyvinyl)-3,3,5,5-tetramethylcyclohexanone; 4-tert.-pentylcyclo-hexanone; 5-cyclohexadecen-1-one; 6,7-dihydro-1,1,2,3,3-pentamethyl-4(5h)-indanone; 8-cyclohexadecen-1-one; 9-cycloheptadecen-1-one; cyclopentadecanone; cyclohexadecanone;
of the cycloaliphatic aldehydes such as e.g. 2-methyl-4-(2,2,6-trimethyl-cyclohexen-1-yl)-2-butenal; 4-(4-hydroxy-4-methylpentyl)-3-cyclohexencarbaldehyde; 4-(4-methyl-3-penten-1-yl)-3-cyclohexencarbaldehyde;
of the cycloaliphatic ketones such as e.g. 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1,2,3,4,5,6,7,8-octahydro-2-naphtalenylmethyl-ketone; methyl-2,6,10-trimethyl-2,5,9-cyclododecatrienylketone; tert.-butyl-(2,4-dimethyl-3-cyclohexen-1-yl)ketone;
of the esters of cyclic alcohols such as e.g. 2-tert-butylcyclohexyl acetate; 4-tert-butylcyclohexyl acetate; 2-tert-pentylcyclohexyl acetate; 4-tert-pentyl-cyclo-hexyl- acetate; 3,3,5-trimethylcyclohexyl acetate; decahydro-2-naphthyl acetate; 2-cyclo-pentylcyclopentylcrotonat; 3-pentyltetrahydro-2h-pyran-4-yl acetate; decahydro-2,5,5,8a-tetramethyl-2-naphthyl acetate; 4,7-methano-3a,4,5,6,7,7a-hexa-hydro-5, or, respectively, 6-indenyl acetate; 4,7-methano-3a,4,5,6,7,7a-hexahydro-5, or, respectively, 6-indenyl propionate; 4,7-methano-3a,4,5,6,7,7a-hexahydro-5, or, respectively, 6-inden-yl isobutyrate; 4,7-methanooctahydro-5, or, respectively 6-indenyl acetate;
of the esters of cycloaliphatic alcohols such as e.g. 1-cyclohexylethyl crotonate;
of the esters of cycloaliphatic carboxylic acids such as e.g. allyl-3-cyclohexyl propionate; allylcyclohexyloxy acetate; cis- and trans-methyldihydro jasmonate; cis- and trans-methyl jasmonate; methyl-2-hexyl-3-oxocyclopentan carboxylate; ethyl-2-ethyl-6,6-dimethyl-2-cyclohexen carboxylate; ethyl-2,3,6,6-tetramethyl-2-cyclohexen carboxylate; ethyl-2-methyl-1,3-dioxolan-2-acetate;
of the araliphatic alcohols such as e.g. benzyl alcohol; 1-phenylethyl alcohol; 3-phenyl propanol; 2-phenyl propanol; 2-phenoxy ethanol; 2,2-dimethyl-3-phenyl propanol; 2,2-dimethyl-3-(3-methylphenyl) propanol; 1,1-dimethyl-2-phenylethyl alcohol; 1,1-dimethyl-3-phenyl propanol; 1-ethyl-1-methyl-3-phenyl propanol; 2-methyl-5-phenyl pentanol; 3-methyl-5-phenyl pentanol; 3-phenyl-2-propen-1-ol; 4-methoxybenzyl alcohol; 1-(4-isopropylphenyl) ethanol;
of the esters of araliphatic alcohols and aliphatic carboxylic acids such as e.g. benzyl acetate; benzyl propionate; benzyl isobutyrate; benzyl isovalerianate; 2-phenylethyl acetate; 2-phenylethyl propionate; 2-phenylethyl isobutyrate; 2-phenyl-ethyl isovalerianate; 1-phenylethyl acetate; alpha-trichlormethylbenzyl acetate; alpha, alpha-dimethylphenylethyl acetate; alpha,alpha-dimethylphenylethyl butyrate; cinnamyl acetate; 2-phenoxyethyl isobutyrate; 4-methoxybenzyl acetate;
of the araliphatic ethers such as e.g. 2-phenylethylmethyl ether; 2-phenylethyl-isoamyl ether; 2-phenylethyl-1-ethoxyethyl ether; phenylacetaldehyddimethyl acetal; phenylacetaldehyddiethyl acetal; hydratropaaldehyddimethyl acetal; phenylacetaldehydglycerin acetal; 2,4,6-trimethyl-4-phenyl-1,3-dioxan; 4,4a,5,9b-tetra-hydroindeno[1,2-d]-m-dioxin; 4,4a,5,9b-tetrahydro-2,4-dimethylindeno[1,2-d]-m-dioxin;
of the aromatic and araliphatic aldehydes such as e.g. benzaldehyde; phenyl acetaldehyde; 3-phenyl propanal; hydratropa aldehyde; 4-methyl benzaldehyde; 4-methylphenyl acetaldehyde; 3-(4-ethylphenyl)-2,2-dimethyl propanal; 2-methyl-3-(4-isopropylphenyl) propanal; 2-methyl-3-(4-isobutylphenyl) propanal; 3-(4-tert.-butyl-phenyl) propanal; cinnamic aldehyde; alpha-butylcinnamic aldehyde; alpha-hexylcinnamic aldehyde; 3-methyl-5-phenyl pentanal; 4-methoxy benzaldehyde; 4-hydroxy-3-methoxy benzaldehyde; 4-hydroxy-3-ethoxy benzaldehyde; 3,4-methylendioxy benzaldehyde; 3,4-dimethoxy benzaldehyde; 2-methyl-3-(4-methoxyphenyl) propanal; 2-methyl-3-(4-methylendioxyphenyl) propanal;
of the aromatic and araliphatic ketones such as e.g. acetophenone; 4-methylacetophenone; 4-methoxyacetophenone; 4-tert.-butyl-2,6-dimethylacetophenone; 4-phenyl-2-butanone; 4-(4-hydroxyphenyl)-2-butanone; 1-(2-naphtha-lenyl) ethanone; 2-benzofuranyl ethanone; (3-methyl-2-benzofuranyl) ethanone; benzophenone; 1,1,2,3,3,6-hexamethyl-5-indanyl methyl ketone; 6-tert.-butyl-1,1-di-methyl-4-indanyl methyl ketone; 1-[2,3-dihydro-1,1,2,6-tetramethyl-3-(1-methylethyl) -1h-5-indenyl] ethanone; 5',6',7',8'-tetrahydro-3',5',5',6',8',8'-hexamethyl-2-aceto-naphthone;
of the aromatic and araliphatic carboxylic acids and their esters such as e.g. benzoic acid; phenyl acetic acid; methyl benzoate; ethyl benzoate; hexyl benzoate; benzyl benzoate; methylphenyl acetate; ethylphenyl acetate; geranylphenyl acetate; phenylethylphenyl acetate; methyl cinnamate; ethyl cinnamate; benzyl cinnamate; phenylethyl cinnamate; cinnamyl cinnamate; allylphenoxy acetate; methyl salicylate; hexyl salicylate; cyclohexyl salicylate; cis-3-hexenyl salicylate; benzyl salicylate; phenylethyl salicylate; methyl-2,4-dihydroxy-3,6-dimethyl benzoate; ethyl-3-phenyl glycidate; ethyl-3-methyl-3-phenyl glycidate;
of the nitrogen containing aromatic compounds such as e.g. 2,4,6-trinitro-1,3-dimethyl-5-tert.-butylbenzol; 3,5-dinitro-2,6-dimethyl-4-tert.-butyl acetophenone; cinnamic acid nitrile; 3-methyl-5-phenyl-2-pentenic acid nitrile; 3-methyl-5-phenyl valeric acid nitrile; methyl anthranilate; methy-n-methyl anthranilate; schiff bases of methyl anthranilate with 7-hydroxy-3,7-dimethyl octanal, 2-methyl-3-(4-tert.-butyl-phenyl) propanal or 2,4-dimethyl-3-cyclohexene carbaldehyde; 6-isopropyl chinolin; 6-isobutyl chinolin; 6-sec.-butyl chinolin; 2-(3-phenylpropyl) pyridine; indol; skatol; 2-methoxy-3-isopropyl pyrazin; 2-isobutyl-3-methoxy pyrazin;
of the phenols, phenyl ethers and phenyl esters such as e.g. estragol; anethol; eugenylmethyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxy benzol; eugenyl acetate; 2-methoxy-4-methyl phenol; 2-ethoxy-5-(1-propenyl) phenol; p-cresyl phenyl acetate;
of the heterocyclic compounds such as e.g. 2,5-dimethyl-4-hydroxy-2h-furan-3-one; 2-ethyl-4-hydroxy-5-methyl-2h-furan-3-one; 3-hydroxy-2-methyl-4h-pyran-4-one; 2-ethyl-3-hydroxy-4h-pyran-4-one; and
of the lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide;4-methyl-1,4-decanolide; 1,15-pentadecanolide; 1,16-hexadeca-nolide; 9-hexadecen-1,16-olide; 10-oxa-1,16-hexadecanolide; 11-oxa-1,16-hexa-decanolide; 12-oxa-1,16-hexadecanolide; ethylen-1,12-dodecandioat; ethylen-1,13-tridecandioat; 2,3-dihydrocoumarin; octahydro coumarin.

Preferred is a fragrance composition according to the invention, wherein the total amount of the compound of formula (epi-I) as defined herein (if present) and of the compound of formula (epi-II) as defined herein (if present) and of the compound of formula (I) as defined herein (if present) and of the compound of formula (II) as defined herein (if present) is in the range of from 0.0001 to 25 wt.-%, preferably of from 0.001 to 10 wt.-%, more preferably of from 0.01 to 5 wt.-%, based on the total weight of the fragrance composition.

According to a preferred embodiment of the fragrance composition of the present invention, the total amount of the compound of formula (epi-I) as defined herein is in the range of from 0.0001 to 25 wt.-%, preferably of from 0.001 to 10 wt.-%, more preferably of from 0.01 to 5 wt.-%, based on the total weight of the fragrance composition.

According to another preferred embodiment of the fragrance composition of the present invention, the total amount of the compound of formula (epi-II) as defined herein is in the range of from 0.0001 to 25 wt.-%, preferably of from 0.001 to 10 wt.-%, more preferably of from 0.01 to 5 wt.-%, based on the total weight of the fragrance composition.

According to another preferred embodiment of the fragrance composition of the present invention, the total amount of the compound of formula (epi-I) as defined herein and of the compound of formula (epi-II) as defined herein is in the range of from 0.0001 to 25 wt.-%, preferably of from 0.001 to 10 wt.-%, more preferably of from 0.01 to 5 wt.-%, based on the total weight of the fragrance composition.

Another preferred embodiment of the present invention is a fragrance composition (as defined herein), wherein the amount of a mixture as defined herein or the amount of the compound of formula (epi-I) as defined herein and/or of the compound of formula (epi-II) as defined herein is sufficient,
(a) to mask or to reduce the or one or several unpleasant odour impression(s) of another fragrance substance in the fragrance composition,
   and/or
(b) to enhance the or one or several pleasant odour impression(s) of another fragrance substance in the fragrance composition.

Another preferred embodiment of the present invention is a fragrance composition (as defined herein), wherein the amount of the compound of formula (epi-I) as defined herein is sufficient, (a) to mask or to reduce the or one or several unpleasant odour impression(s) of another fragrance substance in the fragrance composition, and/or (b) to enhance the or one or several pleasant odour impression(s) of another fragrance substance in the fragrance composition.

Another preferred embodiment of the present invention is a fragrance composition (as defined herein), wherein the amount of the compound of formula (epi-II) as defined herein is sufficient, (a) to mask or to reduce the or one or several unpleasant odour impression(s) of another fragrance substance in the fragrance composition, and/or (b) to enhance the or one or several pleasant odour impression(s) of another fragrance substance in the fragrance composition.

Another preferred embodiment of the present invention is a fragrance composition (as defined herein), wherein the total amount of the compound of formula (epi-I) and of the compound of formula (epi-II) as defined herein is sufficient, (a) to mask or to reduce the or one or several unpleasant odour impression(s) of another fragrance substance in the fragrance composition, and/or (b) to enhance the or one or several pleasant odour impression(s) of another fragrance substance in the fragrance composition.

Another preferred embodiment of the present invention is a fragrance composition (as defined herein), wherein the amount of a mixture as defined herein is sufficient, (a) to mask or to reduce the or one or several unpleasant odour impression(s) of another fragrance substance in the fragrance composition, and/or (b) to enhance the or one or several pleasant odour impression(s) of another fragrance substance in the fragrance composition.

Another aspect of the present invention relates to a perfumed product comprising, preferably in a sensorially effective amount,
(i) the compound of formula (epi-I) as defined herein and/or, preferably and, the compound of formula (epi-II) as defined herein, or
(ii) a mixture as defined herein, or
(iii) a fragrance composition as defined herein,
preferably wherein the total amount of the (i) compound(s) or (ii) mixture or (iii) fragrance composition in the perfumed product is in the range of from 0.01 to 20 wt.-%, more preferably of from 0.03 to 5 wt.-%, most preferably of from 0.05 to 2 wt.-%, based on the total weight of the perfumed product.

Preferably, the product is selected from the group consisting of perfume extracts, eau de parfums, eau de toilettes, aftershaves, eau de colognes, pre-shave products, splash colognes, perfumed refreshing wipes, acidic, alkaline and neutral detergents, textile fresheners, ironing aids, liquid detergents, powdery detergents, laundry pre-treatment agents, fabric softeners, washing soaps, washing tablets, disinfectants, surface disinfectants, air fresheners, aerosol sprays, waxes and polishes, body care products, hand creams and lotions, foot creams and lotions, depilatory creams and lotions, after-shave creams and lotions, tanning creams and lotions, hair care products, deodorants and antiperspirants, decorative cosmetic products, candles, lamp oils, incense sticks, insecticides, repellents, and fuels.

Another aspect of the present invention relates to a method for manufacturing a perfumed product, preferably a perfumed product as defined herein, comprising or consisting of the following steps:
(a) Providing
   (i) the compound of formula (epi-I) as defined herein and/or, preferably and, the compound of formula (epi-II) as defined herein, or
   (ii) a mixture as defined herein, or
   (iii) a fragrance composition as defined herein,
(b) providing one or several further component(s) of the perfumed product to be manufactured, and
(c) contacting or mixing the further component(s) provided in step (b) with a sensorially effective amount of the (i) compound(s) or (ii) mixture or (iii) fragrance composition provided in step (a).

A preferred embodiment of the method for manufacturing a perfumed product, preferably a perfumed product as defined herein, comprises or consists of the following steps:
(a) Providing the compound of formula (epi-I) as defined herein and the compound of formula (epi-II) as defined herein,
(b) providing one or several further component(s) of the perfumed product to be manufactured, and
(c) contacting or mixing the further component(s) provided in step (b) with a sensorially effective amount of the compounds provided in step (a).

Another preferred embodiment of the method for manufacturing a perfumed product, preferably a perfumed product as defined herein, comprises or consists of the following steps:
(a) Providing a mixture as defined herein,
(b) providing one or several further component(s) of the perfumed product to be manufactured, and
(c) contacting or mixing the further component(s) provided in step (b) with a sensorially effective amount of the mixture provided in step (a).

Another preferred embodiment of the method for manufacturing a perfumed product, preferably a perfumed product as defined herein, comprises or consists of the following steps:
(a) Providing a fragrance composition as defined herein,
(b) providing one or several further component(s) of the perfumed product to be manufactured, and
(c) contacting or mixing the further component(s) provided in step (b) with a sensorially effective amount of the fragrance composition provided in step (a).

Another aspect of the present invention relates to the use
(i) of the compound of formula (epi-I) as defined herein and/or, preferably and, of the compound of formula (epi-II) as defined herein, or
(ii) of a mixture as defined herein, or
(iii) of a fragrance composition as defined herein
as a fragrance, preferably for imparting, modifying and/or enhancing one or more odour note(s) selected from the group consisting of woody, ambergris, white amber, flowery, spicy, fruity, musk, animalic, aromatic, aldehydic, and citrus, more preferably selected from the group consisting of woody, ambergris, and white amber.

Preferred is the use of the compound of formula (epi-I) as defined herein as a fragrance, preferably for imparting, modifying and/or enhancing one or more odour note(s) selected from the group consisting of woody, ambergris, white amber, flowery, spicy, fruity, musk, animalic, aromatic, aldehydic, and citrus, more preferably selected from the group consisting of woody, ambergris, and white amber.

Further preferred is the use of the compound of formula (epi-II) as defined herein as a fragrance, preferably for imparting, modifying and/or enhancing one or more odour note(s) selected from the group consisting of woody, ambergris, white amber, flowery, spicy, fruity, musk, animalic, aromatic, aldehydic, and citrus, more preferably selected from the group consisting of woody, ambergris, and white amber.

Further preferred is the use of the compound of formula (epi-I) as defined herein and of the compound of formula (epi-II) as defined herein as a fragrance, preferably for imparting, modifying and/or enhancing one or more odour note(s) selected from the group consisting of woody, ambergris, white amber, flowery, spicy, fruity, musk, animalic, aromatic, aldehydic, and citrus, more preferably selected from the group consisting of woody, ambergris, and white amber.

Further preferred is the use a mixture as defined herein as a fragrance, preferably for imparting, modifying and/or enhancing one or more odour note(s) selected from the group consisting of woody, ambergris, white amber, flowery, spicy, fruity, musk, animalic, aromatic, aldehydic, and citrus, more preferably selected from the group consisting of woody, ambergris, and white amber.

Further preferred is the use a fragrance composition as defined herein as a fragrance, preferably for imparting, modifying and/or enhancing one or more odour note(s) selected from the group consisting of woody, ambergris, white amber, flowery, spicy, fruity, musk, animalic, aromatic, aldehydic, and citrus, more preferably selected from the group consisting of woody, ambergris, and white amber.

Another aspect of the present invention relates to the use
(i) of the compound of formula (epi-I) as defined herein and/or, preferably and, of the compound of formula (epi-II) as defined herein, or
(ii) of a mixture as defined herein, or
(iii) of a fragrance composition as defined herein
to
(a) mask or reduce the or one or several unpleasant odour impression(s) of one or more unpleasantly smelling substance(s),
   and/or
(b) enhance the or one or several pleasant odour impression(s) of one or more pleasantly smelling substance(s), preferably to enhance one or more odour note(s) selected from the group consisting of woody, ambergris, white amber, flowery, spicy, fruity, musk, animalic, aromatic, aldehydic and citrus, more preferably to enhance one or more odour note(s) selected from the group consisting of woody, ambergris, and white amber.

Another aspect of the present invention relates to the use of a perfumed product as defined herein to perfume textiles, hair, skin, surfaces and/or ambient air.

Another aspect of the present invention relates to a method for providing and/or enhancing one or more odour note(s) selected from the group consisting of ambergris, white amber, and woody to/in a product, preferably a perfumed product as defined herein, comprising or consisting of the following steps:
(a) Identifying a product to/in which one or more odour note(s) selected from the group consisting of ambergris, white amber, and woody is to be provided and/or enhanced, preferably a perfumed product as defined herein, and
(b) incorporating
   (i) the compound of formula (epi-I) as defined herein and/or, preferably and, the compound of formula (epi-II) as defined herein, or
   (ii) a mixture as defined herein, or
   (iii) a fragrance composition as defined herein,
   into the product in an amount sufficient to provide and/or enhance one or more odour note(s) selected from the group consisting of ambergris, white amber, and woody to/in the product.

Another aspect of the present invention relates to a method
(a) for enhancing the or one or several pleasant odour impression(s) of one or more pleasantly smelling substance(s),
   and/or
(b) for masking or reducing the or one or several unpleasant odour impression(s) of one or more unpleasantly smelling substance(s),
comprising the following step:
Mixing the (a) pleasantly and/or (b) unpleasantly smelling substance(s) with
(i) the compound of formula (epi-I) as defined herein and/or, preferably and, the compound of formula (epi-II) as defined herein, or
(ii) a mixture as defined herein, or
(iii) a fragrance composition as defined herein,
wherein the amount of (i) compound(s) or (ii) mixture or (iii) fragrance composition is sufficient to (a) enhance the pleasant odour impression(s) of the pleasantly smelling substance(s) and/or (b) to mask or to reduce the unpleasant odour impression(s) of the unpleasantly smelling substance(s).

Another aspect of the present invention relates to a method for producing
(i) the compound of formula (epi-I) as defined herein and/or, preferably and, the compound of formula (epi-II) as defined herein or
(ii) a mixture as defined herein
comprising or consisting of the following steps:
(a) Providing the epi-cis-cedranediol of formula (III) or a mixture comprising the epi-cis-cedranediol of formula (III) 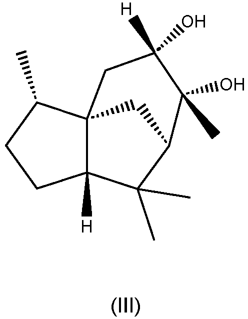
(b) reacting the epi-cis-cedranediol of formula (III) with the compound of formula (IV) (methyl n-propyl ketone, 2-pentanone) 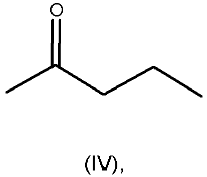 preferably in the presence of one or more water-binding substance(s) and/or of one or more acid(s).

Preferred water-binding substances for use in step (b) are selected from the group consisting of orthoformic acid esters, more preferably orthoformic acid trimethyl ester and orthoformic acid triethylester, and 2,2-dialkoxypentanes, more preferably 2,2-dimethoxypentane and 2,2-diethoxypentane.

Preferred acids for use in step (b) are selected from the group consisting of inorganic protic acids, organic protic acids and fixed-bed acid catalysts.

The epi-cis-cedranediol of formula (III) or a mixture comprising the epi-cis-cedranediol of formula (III) can be obtained by epoxidation and subsequent ring opening from epi-(-)-α-cedrene of formula (V) or from a mixture comprising the epi-(-)-α-cedrene of formula (V). The epi-(-)-α-cedrene of formula (V) or the mixture comprising epi-(-)-α-cedrene of formula (V) in turn may be obtained from natural sources, for example from wood, roots or leaves of plants such junipers, cypresses, sandalwood and cedars or by isomerization of (-)-α-cedrene as described in the literature (Science 2022, 378 (6618), 383-390)

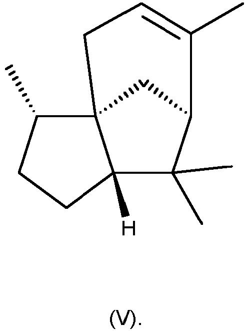

Alternatively, the epi-cis-cedranediol of formula (III) or a mixture comprising the epi-cis-cedranediol of formula (III) can be obtained by dihydroxylation of the epi-(-)-α-cedrene of formula (V) or of a mixture comprising the epi-(-)-α-cedrene of formula (V) with KMnO₄ analogously to the procedures as previously described in WO 2022/223117.

(Preferred) embodiments of the compounds according to the invention correspond to or can be derived from the (preferred) embodiments of the mixtures according to the invention which are explained above or *vice versa.* (Preferred) embodiments of the compounds or mixtures according to the invention correspond to or can be derived from the (preferred) embodiments of the methods according to the invention which are explained above or *vice versa.* (Preferred) embodiments of the compounds or mixtures according to the invention correspond to or can be derived from the (preferred) embodiments of the uses according to the invention which are explained above or *vice versa.* (Preferred) embodiments of the compounds or mixtures according to the invention correspond to or can be derived from the (preferred) embodiments of the fragrance compositions according to the invention which are explained above or *vice versa.* (Preferred) embodiments of the compounds or mixtures according to the invention correspond to or can be derived from the (preferred) embodiments of the perfumed products according to the invention which are explained above or *vice versa.* (Preferred) embodiments of the methods according to the invention correspond to or can be derived from the (preferred) embodiments of the uses according to the invention which are explained above or *vice versa.* Moreover, the (preferred) embodiments described herein can be arbitrarily combined with each other as long as technically sensible.

The invention will now be described in more detail hereinafter with references to the examples. Further aspects of the present invention are disclosed in the accompanying claims. Unless otherwise stated, all values refer to weight-% (wt.-%).

### Examples

### Example 1 - Preparation of a mixture comprising the compounds of formulae (I), (II), (epi-I) and (epi-II)

36 g (0.15 mol) of cis-cedranediol (comprising approximately 1 % of epi-cis-cedranediol), 188 g (2.18 mol) of methyl-n-propyl-ketone, 48.1 g (0.45 mol) of orthoformic acid trimethyl ester and 0.46 g of concentrated sulfuric acid were stirred at 10 °C for 24 hours. At the end of the reaction, methyl-tert-butyl ether was added, it was washed with a 5% sodium hydrogen carbonate solution and the low-boiling components were distilled off. After adding n-hexane, unreacted cis-cedranediol crystallized out, and was separated by filtration. After concentrating the filtrate by evaporation, 45.6 g of raw product were obtained. According to GC analysis, the total content of the compounds of formulae (epi-I), (epi-II), (I) and (II) in the product obtained was 51 % (corresponding to a yield of 50 % of theory). The isomeric mixture obtained was 53.4 % of isomer (I), 45.9 % of isomer (II), 0.1 % isomer (epi-I) and 0.1 % isomer (epi-II).

### Example 2 - Isolation of isomers (epi-I) and (epi-II)

For analytical investigations, the stereoisomeric compounds of formula (epi-I) and (epi-II) were isolated, e.g. from the isomeric mixture obtained in example 1, by preparative gas chromatography (PGC).
PGC conditions: Column: 30 m DB-1 (ID: 0.53 mm, film thickness: 3 µm);
Temperature programme: 100 °C - 240 °C, 6 °C/min;
Carrier gas: Helium
Flow: 5 ml/min
Mass spectrum (epi-I): 306 (3), 291 (4), 263 (36), 221 (100), 203 (63), 133 (25), 119 (34), 105 (22), 91 (13), 69 (25), 43 (27).

Mass spectrum (epi-II): 306 (2), 291 (2), 263 (37), 221 (100), 203 (71), 133 (29), 119 (34), 105 (22), 91 (13), 69 (25), 43 (23).

(epi-I): **¹H NMR (600 MHz,** C₆D₆) δ 4.04 (dd, J = 9.1, 6.6 Hz, 1H), 2.02 (dd, J = 13.5, 6.5 Hz, 1H), 1.97 (d, *J* = 12.2 Hz, 1H), 1.96 (d, *J* = 5.2 Hz, 1H), 1.96 - 1.87 (m, 2H), 1.66 - 1.58 (m, 2H), 1.55 (s, 3H), 1.54 (s, 3H), 1.57 - 1.50 (m, 2H), 1.47 - 1.41 (m, 1H), 1.41 (dt, *J =* 12.2, 1.6 Hz, 1H), 1.36 (ddd, *J =* 11.9, 4.7, 2.6 Hz, 1H), 1.29 - 1.20 (m, 1H), 1.23 - 1.18 (m, 1H), 1.12 (qd, J = 11.8, 6.9 Hz, 1H), 0.97 (s, 3H), 0.95 (t, *J* = 7.4 Hz, 3H), 0.90 (d, *J =* 6.7 Hz, 3H), 0.85 (s, 3H). ¹³C **NMR (151 MHz,** C₆D₆) δ 110.77 (C), 84.98 (C), 78.92 (CH), 57.92 (CH), 56.29 (CH), 54.29 (C), 45.82 (CH₂), 44.45 (CH₂), 43.51 (C), 42.70 (CH), 33.84 (CH₂), 31.94 (CH₂), 31.26 (CH₃), 28.25 (CH₃), 28.02 (CH₃), 27.63 (CH₃), 24.78 (CH₂), 18.78 (CH₂), 14.95 (CH₃), 14.05 (CH₃).

(epi-II): **¹H NMR (600 MHz, C₆D₆)** δ 4.02 (dd, J = 9.1, 6.5 Hz, 1H), 2.06 (dd, J = 13.5, 6.6 Hz, 1H), 1.97 (d, *J* = 12.0 Hz, 1H), 1.96 (d, *J* = 4.6 Hz, 1H), 1.93 - 1.81 (m, 2H), 1.62 (ddd, *J* = 13.4, 9.0, 2.4 Hz, 1H), 1.58 (s, 3H), 1.60 - 1.50 (m, 3H), 1.52 (s, 3H), 1.47 - 1.40 (m, 1H), 1.41 (d, *J =* 11.3 Hz, 1H), 1.38 (ddd, *J =* 12.1, 4.8, 2.6 Hz, 1H), 1.29 - 1.21 (m, 1H), 1.24 - 1.18 (m, 1H), 1.17 - 1.08 (m, 1H), 0.97 (s, 3H), 0.94 (t, *J* = 7.4 Hz, 3H), 0.88 (d, *J* = 6.7 Hz, 3H), 0.86 (s, 3H). **¹³C NMR (151 MHz, C₆D₆)** δ 110.78 (C), 85.14 (C), 78.86 (CH), 57.85 (CH), 56.35 (CH), 54.25 (C), 46.52 (CH₂), 44.51 (CH₂), 43.49 (C), 42.70 (CH), 33.88 (CH₂), 32.00 (CH₂), 31.26 (CH₃), 28.28 (CH₃), 28.00 (CH₃), 27.08 (CH₃), 24.79 (CH₂), 18.95 (CH₂), 14.85 (CH₃), 14.05 (CH₃).

### Example 3 - Sensory analysis

The isomeric mixture obtained in example 1 was compared with Ambrostar^{®} (mixture comprising 53.6 % compound of formula (I) and 46.0 % compound of formula (II)). Surprisingly, the mixture obtained according to example 1 was found to have a more intense woody, ambery profile, especially the desired white-amber notes were more pronounced. As a result, the mixture obtained in example 1 is more powerful and gives the applications a more woody, white-amber character.

### Example 4 - Applications in perfume oils with sensory analysis

### (i) Perfume oils 1A and 1B

| **Ingredient** | **1A** | **1B** |
|---|---|---|
| Undecenal, 2,6,10-trimethyl-9- | 15 | 15 |
| Propanal, 2-methyl-2-(4-ethylbenzyl)- | 3 | 3 |
| Cis-3 hexenyl acetate | 3 | 3 |
| Cyclohexene, 2,4-dimethyl-1-formyl-3-(*E*/*Z*) | 2 | 2 |
| Tricyclo(5.2.1.0)decane, 8-formyl- | 2 | 2 |
| Cyclohexyloxyallyl acetate | 4 | 4 |
| Heptenal, 2,6-dimethyl-5- | 2 | 2 |
| Identoil ^{®} bergamot | 45 | 45 |
| Myrcenol, dihydro- | 90 | 90 |
| 2,6-Octadienal, 3,7-dimethyl-(2E/Z) | 10 | 10 |
| 2-Pentene nitrile, 3-methyl-5-phenyl-(2E/Z) | 3 | 3 |
| Orange oil | 30 | 30 |
| Hexenaldimethylacetal, 2,2,5-trimethyl-4- | 2 | 2 |
| Lavandin oil | 10 | 10 |
| Cardamom oil | 1 | 1 |
| Ethyl pentanoate, 2-methyl-, 10% DPG | 10 | 10 |
| Benzene propanal, alpha-methyl-4-(1,1-dimethylethyl)- | 40 | 40 |
| 2H-1,5-benzodioxepin-3(4H)-one, 7-methyl- | 20 | 20 |
| Benzene propanol, beta,beta.3-trimethyl- | 40 | 40 |
| Geranium oil | 2 | 2 |
| 3-Buten-2-one, 1-(2,4,4-trimethyl-2-cyclohexen-1-yl)-(2E) | 10 | 10 |
| alpha-Hexylcinnamaldehyde | 60 | 60 |
| Cyclopentane methyl acetate, 3-oxo-2-pentyl-(E) | 90 | 90 |
| Propane nitrile, 3-(3-hexenyloxy)-(Z) | 2 | 2 |
| 3-Buten-2-one, 4-(2,6,6-trimethyl-1-cyclohexen-1-yl)- | 5 | 5 |
| Benzene, 1-methoxy-4-(1-propenyl)-(E) | 10 | 10 |
| Ethanone, 1-(2-benzofuranyl)- | 2 | 2 |
| Cyclohexanol acetate, 4-(1,1-dimethylethyl)-(Z) | 30 | 30 |
| Cedarwood Oil | 15 | 15 |
| Cyclohexane propanol, alpha-ethyl-2,2,6-trimethyl-(E/Z) | 30 | 30 |
| Bicyclo[4.4.0]decen, 3,4,10,10-tetramethyl-3-hydroxyethyl-1 (6) | 100 | 100 |
| Cyclododecane, (ethoxymethoxy)- | 30 | 30 |
| Patchouli oil | 10 | 10 |
| 2-Buten-1-ol, 2-ethyl-4-(2,2,3-trimethyl-3-cyclopenten-1-yl)-(2E) | 30 | 30 |
| Methyl benzoate, 2,4-dihydroxy-3,6-dimethyl- | 3 | 3 |
| Tetramethyl dodecahydro-3a,6,6,9a-naphtho(2,1-b)furan | 12 | 12 |
| Isolongifolanone ethanediol ketal | 50 | 50 |
| Pentadecanolide, 1,15- | 30 | 30 |
| Cyclohexadecenone, 8E/Z- | 120 | 120 |
| Dipropylene glycol | 25 | 25 |
| **Ambrostar^{®} (mixture comprising 53.6 % compound of formula (I) and 46.0 % compound of formula (II)), 10.0 % in DPG** | **2** | **0** |
| **Mixture obtained in example 1, 10.0 % in DPG** | **0** | **2** |
| TOTAL | 1000 | 1000 |

By replacing the Ambrostar^{®} in the reference application 1A with the mixture obtained in example 1 (application 1B), the white wood, cedar, amber and sandalwood aspects were particularly enhanced.

### (ii) Perfume oils 2A and 2B

| **Ingredient** | **2A** | **2B** |
|---|---|---|
| Undecenal, 2,6,10-trimethyl-9- | 8 | 8 |
| 10-Undecenal | 8 | 8 |
| Cyclohexene, 2,4-dimethyl-1-formyl-3-(E/Z) | 5 | 5 |
| 3Z-Hexenylmethyl carbonate | 8 | 8 |
| Indeno[1,2-d]-1,3-dioxin, 4.4a.5.9b-tetrahydro-2,4- dimethyl- | 70 | 70 |
| Cyclohexanol acetate, 3,3,5-trimethyl- | 20 | 20 |
| Orange oil | 20 | 20 |
| Methyl Naphthyl Ketone Beta | 5 | 5 |
| Ethanone, 1-(3-methyl-2-benzofuranyl)- | 2 | 2 |
| 2-Butanone, 4-(4-hydroxyphenyl)- | 1 | 1 |
| Peach Base | 8 | 8 |
| Pyranol, 2-isobutyl-4-methyl-4-tetrahydro-(E/Z) | 10 | 10 |
| 1.6-Octadien-3-ol, 3,7-dimethyl- | 90 | 90 |
| 2H-Pyran, tetrahydro-2-methyl-2-(2-methyl-1-propenyl)-(E/Z) | 3 | 3 |
| 2-Phenylethanol | 70 | 70 |
| Benzene pentanol, beta-methyl- | 25 | 25 |
| 2,6-Octadien-1-olacetate, 3,7-dimethyl-(2E/Z) | 30 | 30 |
| But-2-en-1-one, 1-(2,6,6-trimethyl-cyclohex-3-enyl)-(E) | 3 | 3 |
| Propyl benzoate | 10 | 10 |
| Methyl-dihydrojasmonate (E) | 70 | 70 |
| Amyl salicylate N/iso | 17 | 17 |
| Benzyl salicylate | 80 | 80 |
| Cis-3 hexenyl salicylate | 10 | 10 |
| Hexyl benzoate, 2-hydroxy- | 33 | 33 |
| 3-Decen-5-ol, 4-methyl- | 5 | 5 |
| Orris Base | 5 | 5 |
| 3-Buten-2-one, 3-methyl-4-(2,6,6-trimethyl-2-cyclohexen-1-yl)- | 80 | 80 |
| Benzaldehyde, 3,4-methylene dioxy- | 10 | 10 |
| Vanillin | 4 | 4 |
| Cinnamon alcohol | 4 | 4 |
| 2H-1-Benzopyran-2-one | 8 | 8 |
| Acetylcedrene | 70 | 70 |
| Cyclododecane, (ethoxymethoxy)- | 40 | 40 |
| 2-Pentanone, 4-cyclohexyl-4-methyl- | 8 | 8 |
| Patchouli Base | 37 | 37 |
| Patchouli oil | 33 | 33 |
| Pentadecen-1,15-olide, (11E/Z)- + pentadecen-1,15- olide, 12E- | 40 | 40 |
| Triethyl citrate | 30 | 30 |
| **Ambrostar^{®} (mixture comprising 53.6 % compound of formula (I) and 46.0 % compound of formula (II)), 1.0 % in DPG** | **20** | **0** |
| **Mixture obtained in example 1, 1.0 % in DPG** | **0** | **20** |
| TOTAL | 1000 | 1000 |

By replacing Ambrostar^{®} in reference application 2A with the mixture obtained in example 1 (application 2B), the floral chypre type composition became more pleasant, more natural, warmer and more voluminous in character. In addition, adhesion was improved.

### (iii) Perfume oils 3A and 3B

| **Ingredient** | **3A** | **3B** |
|---|---|---|
| NAPHTHO[2.1-B]FURAN, DODECAHYDRO-3A.6.6.9A-TETRAMETHYL- | 5 | 5 |
| BENZYL SALICYLATE | 50 | 50 |
| BERGAMOT OIL | 50 | 50 |
| COUMARIN | 5 | 5 |
| DAMASCONE, TRANS-ALPHA-, 1% in DPG | 20 | 20 |
| DECALACTONE GAMMA, 10% in DPG | 15 | 15 |
| ETHANEDIOL BRASSYLATE | 30 | 30 |
| NONADIEN-3-OL, 3,7-DIMETHYL-1,6- | 60 | 60 |
| 3,7-DIME-1,6-NONADIEN-3-YL ACETATE | 50 | 50 |
| PYRANOL, 2-ISOBUTYL-4-METHYL-4-TETRAHYDRO-(E/Z) | 70 | 70 |
| 5-HEXENYL BUTYRATE, 2-METHYL- | 5 | 5 |
| BICYCLO[2.2.2]OCTENE, 6-ME-8-ISOPROPYL-2 (3)-(1,3-DIOXOLAN-2-YL | 5 | 5 |
| METHYL-DIHYDROJASMONATE | 280 | 280 |
| PROPANAL, 2-PIPERONYL- | 20 | 20 |
| BENZALDEHYDE, 3,4-METHYLENE DIOXY- | 10 | 10 |
| 2-(1-(3,3-DIMETHYLCYCLOHEXYL)-ETHOXY-2-METHYLPROPYL PROPIONATE | 60 | 60 |
| 3Z-HEXENYL SALICYLATE | 5 | 5 |
| BICYCLO[4.4.0]DECENE, 3,4,10,10-TETRAME-3-HYDROXYETHYL-1(6) | 120 | 120 |
| PENTADECANOLIDE, 1,15- | 30 | 30 |
| MANDARIN OIL | 20 | 20 |
| CYCLOPENTADECENONE, 3-METHYL-5E- | 3 | 3 |
| BUTANOL, 2-METHYL-4-PHENYL-2- | 10 | 10 |
| PYRIDINE, 4-DECYL-, 1% in DPG | 2 | 2 |
| PENTEN-2-OL, 3,3-DIMETHYL-5-(2,2,3-TRIMETHYL-3- CYCLOPENTENYL)-4- | 20 | 20 |
| CYCLOHEXANOL, 4-(3-METHYL-BUTYL)-(E/Z) | 15 | 15 |
| VANILLIN | 20 | 20 |
| DIPROPYLENE GLYCOL | 15 | 15 |
| **Ambrostar^{®} (mixture comprising 53.6 % compound of formula (I) and 46.0 % compound of formula (II)), 0.1 % in DPG** | **5** | **0** |
| **Mixture obtained in example 1, 0.1 % in DPG** | **0** | **5** |
| TOTAL | 1000 | 1000 |

By replacing Ambrostar^{®} in reference application 3A with the mixture obtained in example 1 (application 3B), the whole composition had more volume, was more complex, elegant and therefore of higher quality, and the coumarin note was very well harmonised.

### (iv) Perfume oils 4A and 4B

| **Ingredient** | **4A** | **4B** |
|---|---|---|
| NAPHTHO[2.1-B]FURAN, DODECAHYDRO-3A.6.6.9A-TETRAMETHYL- | 5 | 5 |
| BENZOIC SIAM ABS., 50% in DPG | 10 | 10 |
| BENZYL SALICYLATE | 20 | 20 |
| BERGAMOT OIL | 50 | 50 |
| CEDRYLMETHYL ETHER | 40 | 40 |
| CISTUS LABDANUM | 35 | 35 |
| CISTUS OIL SPAN., 10% in DPG | 10 | 10 |
| DAMASCONE, TRANS-ALPHA-, 1% in DPG | 20 | 20 |
| MYRCENOL, DIHYDRO- | 30 | 30 |
| ETHANEDIOL BRASSYLATE | 120 | 120 |
| METHYL BENZOATE, 2,4-DIHYDROXY-3,6-DIMETHYL-, 1% in DPG | 20 | 20 |
| PYRANOL, 2-ISOBUTYL-4-METHYL-4-TETRAHYDRO-(E/Z) | 20 | 20 |
| PENTADECEN-1,15-OLIDE, (11E/Z)- + PENTADECEN-1,15-OLIDE, 12E- | 30 | 30 |
| METHYL-DIHYDROJASMONATE | 300 | 300 |
| BICYCLO[4.4.0]DECENE, 3,4,10,10-TETRAMETHYL-3- HYDROXYETHYL-1 (6) | 75 | 75 |
| ISOBUTYLQUINOLINE, 10% in DPG | 3 | 3 |
| CYCLOHEXANONE, 3,3,5,5-TETRAMETHYL-4-(1-ETHOXYVINYL)- | 30 | 30 |
| LABDANUM ABSOLUTE | 20 | 20 |
| LINALYL ACETATE | 50 | 50 |
| MANDARIN OIL | 10 | 10 |
| CYCLOPENTADECENONE, 3-METHYL-5E- | 10 | 10 |
| MUSCATEL SAGE OIL | 10 | 10 |
| OLIBANUM COEUR, 50% in TEC | 5 | 5 |
| PATCHOULI OIL | 20 | 20 |
| PYRIDINE, 4-DECYL-, 1% in DPG | 2 | 2 |
| BUTENOL, 2-ETHYL-4-(2,2,3-TRIMETHYL-3-CYCLOPENTENYL)-2E- | 5 | 5 |
| VANILLIN | 30 | 30 |
| **Ambrostar^{®} (mixture comprising 53.6 % compound of formula (I) and 46.0 % compound of formula (II)), 0.1 % in DPG** | **20** | **0** |
| **Mixture obtained in example 1, 0.1 % in DPG** | **0** | **20** |
| TOTAL | 1000 | 1000 |

Replacing Ambrostar^{®} in the reference application 4A with the mixture obtained in example 1 (application 4B) intensified the fine woody and tobacco notes and gave the composition more volume.

### Example 5 - Applications in formulations

The perfume oils 1A, 1B, 2A, 2B, 3A, 3B, 4A and 4B as described above (cf. example 4) were separately incorporated into the following formulations. The olfactory effects described above for each perfume oil were also observed in the following formulations.

### Shower Gel

| **Material** | **INCI name** | **wt. %** | **wt. %** |
|---|---|---|---|
| Deionized water | Water | to 100 | to 100 |
| Plantacare PS 10 | Sodium laureth sulfate, lauryl glucoside | 20.0 | 20.0 |
| Dragocid Liquid | Phenoxyethanol, methylparaben, ethylparaben, butylparaben, propylparaben, isobutylparaben | 0.5 | 0.5 |
| Sodium chloride | Sodium chloride | 1.4 | 1.4 |
| Citric acid monohydrate crystalline | Citric acid | 1.3 | 1.3 |
| **Perfume oil 1A, 1B, 2A, 2B, 3A, 3B, 4A or 4B** | **Perfume (fragrance)** | **0.5** | **0.7** |

### Liquid Detergent

| **Ingredients** | **wt- %** |
|---|---|
| Deionized water | 39.9 |
| Optical brightener | 0.10 |
| Coconut fatty acids (C12-C18) | 7.5 |
| Potassium hydroxide 50% solution | 4.3 |
| Propane-1,2-diol | 5.00 |
| Fatty alcohols C12-C15, 8 EO | 12.0 |
| Na-salt of secondary alkylsulphonates (C13-C17) | 17.0 |
| Triethanolamine | 2.0 |
| Trisodium citrate dihydrate | 5.0 |
| Dequest 2066([[(phosphonomethyl)imino]bis[(ethylenenitrilo)bis(methylene)]]tetrakis-phosphonic acid, sodium salt) | 3.0 |
| Ethanol | 3.0 |
| Enzymes | 0.7 |
| **Perfume oil 1A, 1B, 2A, 2B, 3A, 3B, 4A or 4B** | **0.5** |

### Washing Powder

| **Material** | **Chemical name** | **Function** | **wt. %** | **wt. %** |
|---|---|---|---|---|
| Sodium metasilicate pentahydrate | Sodium metasilicate pentahydrate | | to 100 | to 100 |
| Sodium hydrogen carbonate | Sodium hydrogen carbonate | Alkali | 15.0 | 15.0 |
| Sodium percarbonate | Sodium carbonate peroxyhydrate | Bleach | 15.0 | 15.0 |
| Peractive AC Blue | TAED/Na-carboxymethylcellulose | Activator | 5.00 | 5.00 |
| Genapol OA-080 | Oxoalcohol C14-15, 8EO | Non-ionic surfactant | 3.00 | 3.00 |
| Texapon K12 powder | Sodium lauryl sulfate C12 | Anionic surfactant | 7.00 | 7.00 |
| Tinopal CBS-X | | Brightener | 0.50 | 0.50 |
| Savinase 6.0 T, Type W | Protease | Enzyme | 0.40 | 0.40 |
| Termamyl 120 T | Alpha-amylase | Enzyme | 0.30 | 0.30 |
| Sodium sulfate | Sodium sulfate | Filler | 5.50 | 5.50 |
| **Perfume oil 1A, 1B, 2A, 2B, 3A, 3B, 4A or 4B** | | **Perfume (fragrance)** | **0.30** | **0.50** |

## Claims

1. Compound of formula (epi-I) 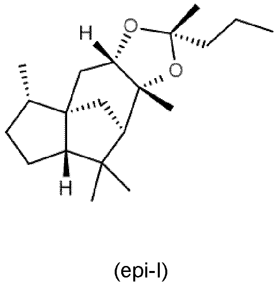 or compound of formula (epi-II)

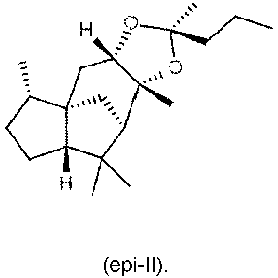

2. Mixture comprising or consisting of the compound of formula (epi-I) as defined in claim 1 and/or the compound of formula (epi-II) as defined in claim 1
and optionally at least one compound selected from the group consisting of the compounds of formula (I) and (II)

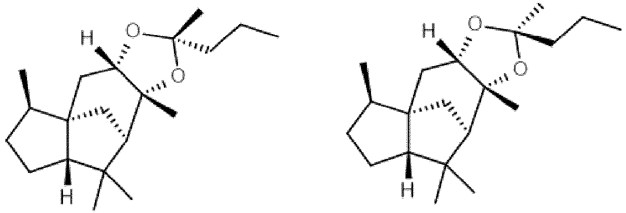

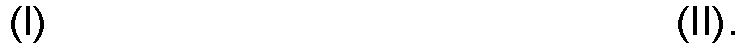

3. Mixture according to claim 2, comprising or consisting of the compound formula (epi-I) as defined in claim 1, and the compound of formula (epi-II) as defined in claim 1, and the compound of formula (I) as defined in claim 2, and the compound of formula (II) as defined in claim 2.

4. Mixture according to claim 2 or 3, wherein the weight ratio of the total amount of the compound of formula (epi-I) as defined in claim 1 and of the compound of (epi-II) as defined in claim 1 to the total amount of the compound of formula (I) as defined in claim 2 and of the compound of formula (II) as defined in claim 2 is from about 0.01:99.99 to about 5:95, preferably from about 0.05:99.95 to about 4:96, more preferably from about 0.10:99.90 to about 3:97.

5. Fragrance composition, preferably perfume oil, comprising or consisting of
(i) the compound of formula (epi-I) as defined in claim 1 and/or the compound of formula (epi-II) as defined in claim 1
or
(ii) a mixture according to any of the claims 2 to 4
and
(iii) one or more additional fragrance substance(s).

6. Fragrance composition according to claim 5, wherein the total amount of the compound of formula (epi-I) as defined in claim 1 and of the compound of formula (epi-II) as defined in claim 1 and of the compound of formula (I) as defined in claim 2 and of the compound of formula (II) as defined in claim 2 is in the range of from 0.0001 to 25 wt.-%, preferably of from 0.001 to 10 wt.-%, more preferably of from 0.01 to 5 wt.-%, based on the total weight of the fragrance composition.

7. Fragrance composition according to claim 5 or 6, wherein the amount of mixture according to any of the claims 2 to 4 or the amount of the compound of formula (epi-I) as defined in claim 1 and/or of the compound of formula (epi-II) as defined in claim 1 is sufficient,
(a) to mask or to reduce the or one or several unpleasant odour impression(s) of another fragrance substance in the fragrance composition,
and/or
(b) to enhance the or one or several pleasant odour impression(s) of another fragrance substance in the fragrance composition.

8. Perfumed product comprising, preferably in a sensorially effective amount,
(i) the compound of formula (epi-I) as defined in claim 1 and/or the compound of formula (epi-II) as defined in claim 1, or
(ii) a mixture according to any of the claims 2 to 4, or
(iii) a fragrance composition according to any of the claims 5 to 7,
preferably wherein the total amount of the (i) compound(s) or (ii) mixture or (iii) fragrance composition in the perfumed product is in the range of from 0.01 to 20 wt.-%, more preferably of from 0.03 to 5 wt.-%, most preferably of from 0.05 to 2 wt.-%, based on the total weight of the perfumed product.

9. Perfumed product according to claim 8, wherein the product is selected from the group consisting of perfume extracts, eau de parfums, eau de toilettes, aftershaves, eau de colognes, pre-shave products, splash colognes, perfumed refreshing wipes, acidic, alkaline and neutral detergents, textile fresheners, ironing aids, liquid detergents, powdery detergents, laundry pre-treatment agents, fabric softeners, washing soaps, washing tablets, disinfectants, surface disinfectants, air fresheners, aerosol sprays, waxes and polishes, body care products, hand creams and lotions, foot creams and lotions, depilatory creams and lotions, after-shave creams and lotions, tanning creams and lotions, hair care products, deodorants and antiperspirants, decorative cosmetic products, candles, lamp oils, incense sticks, insecticides, repellents, and fuels.

10. Method for manufacturing a perfumed product, preferably a perfumed product according to claim 8 or 9, comprising or consisting of the following steps:
(a) Providing
(i) the compound of formula (epi-I) as defined in claim 1 and/or the compound of formula (epi-II) as defined in claim 1, or
(ii) a mixture according to any of the claims 2 to 4, or
(iii) a fragrance composition according to any of the claims 5 to 7,
(b) providing one or several further component(s) of the perfumed product to be manufactured, and
(c) contacting or mixing the further component(s) provided in step (b) with a sensorially effective amount of the (i) compound(s) or (ii) mixture or (iii) fragrance composition provided in step (a).

11. Use
(i) of the compound of formula (epi-I) as defined in claim 1 and/or of the compound of formula (epi-II) as defined in claim 1, or
(ii) of a mixture according to any of the claims 2 to 4, or
(iii) of a fragrance composition according to any of the claims 5 to 7
as a fragrance, preferably for imparting, modifying and/or enhancing one or more odour note(s) selected from the group consisting of woody, ambergris, white amber, flowery, spicy, fruity, musk, animalic, aromatic, aldehydic, and citrus.

12. Use
(i) of the compound of formula (epi-I) as defined in claim 1 and/or of the compound of formula (epi-II) as defined in claim 1, or
(ii) of a mixture according to any of the claims 2 to 4, or
(iii) of a fragrance composition according to any of the claims 5 to 7 to
(a) mask or reduce the or one or several unpleasant odour impression(s) of one or more unpleasantly smelling substance(s),
and/or
(b) enhance the or one or several pleasant odour impression(s) of one or more pleasantly smelling substance(s), preferably to enhance one or more odour note(s) selected from the group consisting of woody, ambergris, white amber, flowery, spicy, fruity, musk, animalic, aromatic, aldehydic and citrus.

13. Use of a perfumed product according to claim 8 or 9 to perfume textiles, hair, skin, surfaces and/or ambient air.

14. Method for providing and/or enhancing one or more odour note(s) selected from the group consisting of ambergris, white amber, and woody to/in a product, preferably a perfumed product as defined in claim 8 or 9, comprising or consisting of the following steps:
(a) Identifying a product to/in which one or more odour note(s) selected from the group consisting of ambergris, white amber, and woody is to be provided and/or enhanced, preferably a perfumed product as defined in claim 8 or 9, and
(b) incorporating
(i) the compound of formula (epi-I) as defined in claim 1 and/or the compound of formula (epi-II) as defined in claim 1, or
(ii) a mixture according to any of the claims 2 to 4, or
(iii) a fragrance composition according to any of the claims 5 to 7,
into the product in an amount sufficient to provide and/or enhance one or more odour note(s) selected from the group consisting of ambergris, white amber, and woody to/in the product.

15. Method
(a) for enhancing the or one or several pleasant odour impression(s) of one or more pleasantly smelling substance(s),
and/or
(b) for masking or reducing the or one or several unpleasant odour impression(s) of one or more unpleasantly smelling substance(s),
comprising the following step:
Mixing the (a) pleasantly and/or (b) unpleasantly smelling substance(s) with
(i) the compound of formula (epi-I) as defined in claim 1 and/or the compound of formula (epi-II) as defined in claim 1, or
(ii) a mixture according to any of the claims 2 to 4, or
(iii) a fragrance composition according to any of the claims 5 to 7,
wherein the amount of (i) compound(s) or (ii) mixture or (iii) fragrance composition is sufficient to (a) enhance the pleasant odour impression(s) of the pleasantly smelling substance(s) and/or (b) to mask or to reduce the unpleasant odour impression(s) of the unpleasantly smelling substance(s).

## Patentansprüche

1. Verbindung der Formel (epi-I) 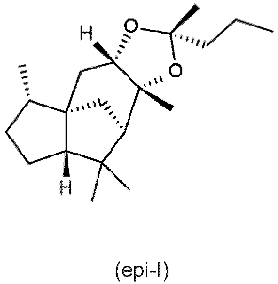 oder Verbindung der Formel (epi-II)

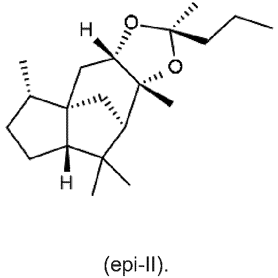

2. Mischung, umfassend oder bestehend aus der Verbindung der Formel (epi-I) wie in Anspruch 1 definiert und/oder der Verbindung der Formel (epi-II) wie in Anspruch 1 definiert
und gegebenenfalls mindestens einer Verbindung ausgewählt aus der Gruppe bestehend aus den Verbindungen der Formel (I) und (II)

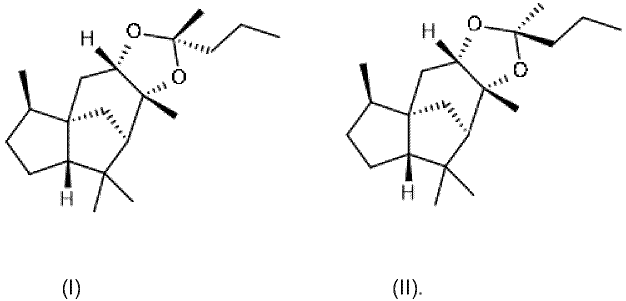

3. Mischung gemäß Anspruch 2, umfassend oder bestehend aus der Verbindung der Formel (epi-I) wie in Anspruch 1 definiert, und der Verbindung der Formel (epi-II) wie in Anspruch 1 definiert und der Verbindung der Formel (I) wie in Anspruch 2 definiert und der Verbindung der Formel (II) wie in Anspruch 2 definiert.

4. Mischung gemäß Anspruch 2 oder 3, wobei das Gewichtsverhältnis der Gesamtmenge der Verbindung der Formel (epi-I) wie in Anspruch 1 definiert und der Verbindung der Formel (epi-II) wie in Anspruch 1 definiert zu der Gesamtmenge der Verbindung der Formel (I) wie in Anspruch 2 definiert und der Verbindung der Formel (II) wie in Anspruch 2 definiert etwa 0,01:99,99 bis etwa 5:95, vorzugsweise etwa 0,05:99,95 bis etwa 4:96, mehr bevorzugt etwa 0,10:99,90 bis etwa 3:97, beträgt.

5. Riechstoffzusammensetzung, vorzugsweise Parfümöl, umfassend oder bestehend aus
(i) der Verbindung der Formel (epi-I) wie in Anspruch 1 definiert und/oder der Verbindung der Formel (epi-II) wie in Anspruch 1 definiert
oder
(ii) einer Mischung nach einem der Ansprüche 2 bis 4
und
(iii) einem oder mehreren zusätzlichen Riechstoff(en).

6. Riechstoffzusammensetzung gemäß Anspruch 5, wobei die Gesamtmenge der Verbindung der Formel (epi-I) wie in Anspruch 1 definiert und der Verbindung der Formel (epi-II) wie in Anspruch 1 definiert und der Verbindung der Formel (I) wie in Anspruch 2 definiert und der Verbindung der Formel (II) wie in Anspruch 2 definiert im Bereich von 0,0001 bis 25 Gew.-%, vorzugsweise von 0,001 bis 10 Gew.-%, mehr bevorzugt von 0,01 bis 5 Gew.-%, bezogen auf das Gesamtgewicht der Riechstoffzusammensetzung, liegt.

7. Riechstoffzusammensetzung gemäß Anspruch 5 oder 6, wobei die Menge der Mischung nach einem der Ansprüche 2 bis 4 oder die Menge der Verbindung der Formel (epi-I) wie in Anspruch 1 definiert und/oder der Verbindung der Formel (epi-II) wie in Anspruch 1 definiert ausreicht,
(a) den oder einen oder mehrere unangenehme(n) Geruchseindruck/-eindrücke einer anderen Riechstoffsubstanz in der Riechstoffzusammensetzung zu maskieren oder zu reduzieren,
und/oder
(b) den oder einen oder mehrere angenehme(n) Geruchseindruck/-eindrücke einer anderen Riechstoffsubstanz in der Riechstoffzusammensetzung zu verstärken.

8. Parfümiertes Produkt, umfassend, vorzugsweise in einer sensorisch wirksamen Menge,
(i) die Verbindung der Formel (epi-I) wie in Anspruch 1 definiert und/oder die Verbindung der Formel (epi-II) wie in Anspruch 1 definiert, oder
(ii) eine Mischung nach einem der Ansprüche 2 bis 4, oder
(iii) eine Riechstoffzusammensetzung nach einem der Ansprüche 5 bis 7,
wobei vorzugsweise die Gesamtmenge der (i) Verbindung(en) oder (ii) Mischung oder (iii) Riechstoffzusammensetzung in dem parfümierten Produkt im Bereich von 0,01 bis 20 Gew.-%, mehr bevorzugt von 0,03 bis 5 Gew.-%, am meisten bevorzugt von 0,05 bis 2 Gew.-%, bezogen auf das Gesamtgewicht des parfümierten Produkts, liegt.

9. Parfümiertes Produkt gemäß Anspruch 8, wobei das Produkt ausgewählt ist aus der Gruppe bestehend aus Parfümextrakten, Eau de Parfums, Eau de Toilettes, Aftershaves, Eau de Colognes, Pre-Shave-Produkten, Splash Colognes, parfümierten Erfrischungstüchern, sauren, alkalischen und neutralen Waschmitteln, Textilerfrischern, Bügelhilfen, Flüssigwaschmitteln, Pulverwaschmitteln, Wäschevorbehandlungsmitteln, Weichspülern, Waschseifen, Waschtabletten, Desinfektionsmitteln, Flächendesinfektionsmitteln, Lufterfrischern, Aerosolsprays, Wachsen und Polituren, Körperpflegeprodukten, Handcremes und -lotionen, Fußcremes und -lotionen, Enthaarungscremes und -lotionen, After-Shave-Cremes und -lotionen, Bräunungscremes und -lotionen, Haarpflegeprodukten, Deodorants und Antitranspirantien, dekorativen kosmetischen Produkten, Kerzen, Lampenölen, Räucherstäbchen, Insektiziden, Repellentien und Brennstoffen.

10. Verfahren zur Herstellung eines parfümierten Produkts, vorzugsweise eines parfümierten Produkts gemäß Anspruch 8 oder 9, umfassend oder bestehend aus den folgenden Schritten:
(a) Bereitstellen
(i) der Verbindung der Formel (epi-I) wie in Anspruch 1 definiert und/oder der Verbindung der Formel (epi-II) wie in Anspruch 1 definiert, oder
(ii) einer Mischung nach einem der Ansprüche 2 bis 4, oder
(iii) einer Riechstoffzusammensetzung nach einem der Ansprüche 5 bis 7,
(b) Bereitstellen eines oder mehrerer weiterer Bestandteile(s) des herzustellenden parfümierten Produkts, und
(c) Inkontaktbringen oder Mischen des/der in Schritt (b) bereitgestellten weiteren Bestandteile(s) mit einer sensorisch wirksamen Menge der in Schritt (a) bereitgestellten (i) Verbindung(en) oder (ii) Mischung oder (iii) Riechstoffzusammensetzung.

11. Verwendung
(i) der Verbindung der Formel (epi-I) wie in Anspruch 1 definiert und/oder der Verbindung der Formel (epi-II) wie in Anspruch 1 definiert, oder
(ii) einer Mischung nach einem der Ansprüche 2 bis 4, oder
(iii) einer Riechstoffzusammensetzung nach einem der Ansprüche 5 bis 7
als Riechstoff, vorzugsweise zum Verleihen, Modifizieren und/oder Verstärken einer oder mehrerer Geruchsnote(n) ausgewählt aus der Gruppe bestehend aus holzig, Ambra, Weißer Amber, blumig, würzig, fruchtig, Moschus, animalisch, aromatisch, aldehydisch und Zitrus.

12. Verwendung
(i) der Verbindung der Formel (epi-I) wie in Anspruch 1 definiert und/oder der Verbindung der Formel (epi-II) wie in Anspruch 1 definiert, oder
(ii) einer Mischung nach einem der Ansprüche 2 bis 4, oder
(iii) einer Riechstoffzusammensetzung nach einem der Ansprüche 5 bis 7
zum
(a) Maskieren oder Reduzieren des oder eines oder mehrerer unangenehmen/unangenehmer Geruchseindrucks/Geruchseindrücke einer oder mehrerer unangenehm riechenden/riechender Substanz(en),
und/oder
(b) Verstärken des oder eines oder mehrerer angenehmen/angenehmer Geruchseindrucks/Geruchseindrücke einer oder mehrerer angenehm riechenden/riechender Substanz(en), vorzugsweise zum Verstärken einer oder mehrerer Geruchsnote(n) ausgewählt aus der Gruppe bestehend aus holzig, Ambra, Weißer Amber, blumig, würzig, fruchtig, Moschus, animalisch, aromatisch, aldehydisch und Zitrus.

13. Verwendung eines parfümierten Produkts gemäß Anspruch 8 oder 9 zum Parfümieren von Textilien, Haar, Haut, Oberflächen und/oder Umgebungsluft.

14. Verfahren zum Bereitstellen und/oder Verstärken einer oder mehrerer Geruchsnote(n) ausgewählt aus der Gruppe bestehend aus Ambra, Weißer Amber und holzig in einem/für ein Produkt, vorzugsweise ein parfümiertes Produkt wie in Anspruch 8 oder 9 definiert, umfassend oder bestehend aus den folgenden Schritten:
(a) Identifizieren eines Produkts, in dem/ für das eine oder mehrere Geruchsnote(n) ausgewählt aus der Gruppe bestehend aus Ambra, Weißer Amber und holzig bereitgestellt und/oder verstärkt werden soll(en), vorzugsweise eines parfümierten Produkts wie in Anspruch 8 oder 9 definiert, und
(b) Einarbeiten
(i) der Verbindung der Formel (epi-I) wie in Anspruch 1 definiert und/oder der Verbindung der Formel (epi-II) wie in Anspruch 1 definiert, oder
(ii) einer Mischung nach einem der Ansprüche 2 bis 4, oder
(iii) einer Riechstoffzusammensetzung nach einem der Ansprüche 5 bis 7,
in das Produkt in einer Menge, die ausreicht, um eine oder mehrere Geruchsnote(n) ausgewählt aus der Gruppe bestehend aus Ambra, Weißer Amber und holzig in dem/für das Produkt bereitzustellen und/oder zu verstärken.

15. Verfahren
(a) zum Verstärken des oder eines oder mehrerer angenehmen/angenehmer Geruchseindrucks/Geruchseindrücke einer oder mehrerer angenehm riechenden/riechender Substanz(en),
und/oder
(b) zum Maskieren oder Reduzieren des oder eines oder mehrerer unangenehmen/unangenehmer Geruchseindrucks/Geruchseindrücke einer oder mehrerer unangenehm riechenden/riechender Substanz(en),
umfassend den folgenden Schritt:
Mischen der (a) angenehm und/oder (b) unangenehm riechenden Substanz(en) mit
(i) der Verbindung der Formel (epi-I) wie in Anspruch 1 definiert und/oder der Verbindung der Formel (epi-II) wie in Anspruch 1 definiert, oder
(ii) einer Mischung nach einem der Ansprüche 2 bis 4, oder
(iii) einer Riechstoffzusammensetzung nach einem der Ansprüche 5 bis 7,
wobei die Menge der (i) Verbindung(en) oder (ii) Mischung oder (iii) Riechstoffzusammensetzung ausreicht, um (a) den/die angenehmen Geruchseindruck/Geruchseindrücke der angenehm riechenden Substanz(en) zu verstärken und/oder (b) den/die unangenehmen Geruchseindruck/Geruchseindrücke der unangenehm riechenden Substanz(en) zu maskieren oder zu reduzieren.

## Revendications

1. Composé de formule (epi-I) 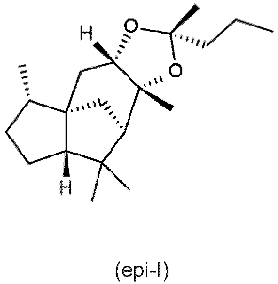 ou composé de formule (épi-II)

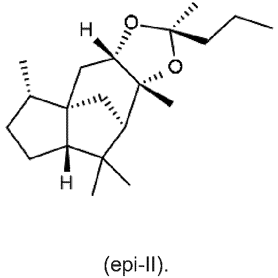

2. Mélange comprenant le ou constitué du composé de formule (épi-I) tel que défini dans la revendication 1 et/ou du composé de formule (épi-II) tel que défini dans la revendication 1
et le cas échéant au moins un composé choisi dans le groupe constitué par les composés de formule (I) et (II)

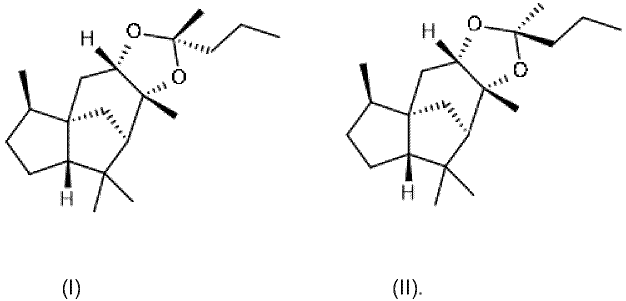

3. Mélange selon la revendication 2, comprenant le ou constitué du composé de formule (épi-I) tel que défini dans la revendication 1, et du composé de formule (épi-II) tel que défini dans la revendication 1, et du composé de formule (I) tel que défini dans la revendication 2 et du composé de formule (II) tel que défini dans la revendication 2.

4. Mélange selon la revendication 2 ou 3, dans lequel le rapport pondéral de la quantité totale du composé de formule (épi-I) tel que défini dans la revendication 1 et du composé (épi-II) tel que défini dans la revendication 1 à la quantité totale du composé de formule (I) tel que défini dans la revendication 2 et du composé de formule (II) tel que défini dans la revendication 2 est compris entre environ 0,01 : 99,99 et environ 5 : 95, de préférence entre environ 0,05 : 99,95 et environ 4 : 96, plus particulièrement entre environ 0,10 : 99,90 et environ 3 : 97.

5. Composition de substance parfumante, de préférence huile de parfum, comprenant ou constituée:
(i) du composé de formule (épi-I) tel que défini dans la revendication 1 et/ou du composé de formule (épi-II) tel que défini dans la revendication 1
ou
(ii) d'un mélange selon l'une quelconque des revendications 2 à 4
et
(iii) d'une ou de plusieurs substance(s) parfumante(s) supplémentaire(s).

6. Composition de substance parfumante selon la revendication 5, dans laquelle la quantité totale du composé de formule (épi-I) tel que défini dans la revendication 1 et du composé de formule (épi-II) tel que défini dans la revendication 1 et du composé de formule (I) tel que défini dans la revendication 2 et du composé de formule (II) tel que défini dans la revendication 2 est comprise entre 0,0001 et 25 % en poids, de préférence entre 0,001 et 10 % en poids, plus particulièrement entre 0,01 et 5 % en poids, par rapport au poids total de la composition de substance parfumante.

7. Composition de substance parfumante selon la revendication 5 ou 6, dans laquelle la quantité du mélange selon l'une quelconque des revendications 2 à 4 ou la quantité du composé de formule (épi-I) tel que défini dans la revendication 1 et/ou du composé de formule (épi-II) tel que défini dans la revendication 1 est suffisante
(a) pour masquer ou réduire la ou une ou plusieurs impression(s) olfactive(s) désagréable(s) d'une autre substance parfumante dans la composition de substance parfumante,
et/ou
(b) pour intensifier la ou une ou plusieurs impression(s) olfactive(s) agréables d'une autre substance parfumante dans la composition de substance parfumante.

8. Produit parfumé comprenant, de préférence en une quantité sensoriellement efficace:
(i) le composé de formule (épi-I) tel que défini dans la revendication 1 et/ou le composé de formule (épi-II) tel que défini dans la revendication 1, ou
(ii) un mélange selon l'une quelconque des revendications 2 à 4, ou
(iii) une composition de substance parfumante selon l'une quelconque des revendications 5 à 7,
de préférence dans lequel la quantité totale du (des) (i) composé(s) ou du (ii) mélange ou de la (iii) composition de substance parfumante dans le produit parfumé est comprise entre 0,01 et 20 % en poids, plus particulièrement entre 0,03 et 5 % en poids, de manière la plus préférée entre 0,05 et 2 % en poids, par rapport au poids total du produit parfumé.

9. Produit parfumé selon la revendication 8, dans lequel le produit est choisi dans le groupe constitué par les extraits de parfum, les eaux de parfum, les eaux de toilette, les après-rasages, les eaux de Cologne, les produits de pré-rasage, les eaux de Cologne splash, les lingettes rafraîchissantes parfumées, les détergents acides, alcalins et neutres, les assainisseurs de textiles, les aides au repassage, les détergents liquides, les détergents en poudre, les agents de prétraitement du linge, les assouplissants, les savons de lavage, les pastilles de lavage, les désinfectants, les désinfectants de surface, les assainisseurs d'air, les sprays aérosols, les cires et les produits de cirage, les produits de soins personnels, les crèmes et lotions pour les mains, les crèmes et lotions pour les pieds, les crèmes et lotions dépilatoires, les crèmes et lotions après-rasage, les crèmes et lotions de bronzage, les produits de soins capillaires, les déodorants et les antiperspirants, les produits cosmétiques décoratifs, les bougies, les huiles de lampe, les bâtons d'encens, les insecticides, les répulsifs et les carburants.

10. Procédé de production d'un produit parfumé, de préférence d'un produit parfumé selon la revendication 8 ou 9, comprenant ou consistant en les étapes suivantes:
(a) fournir
(i) le composé de formule (épi-I) tel que défini dans la revendication 1 et/ou le composé de formule (épi-II) tel que défini dans la revendication 1, ou
(ii) un mélange selon l'une quelconque des revendications 2 à 4, ou
(iii) une composition de substance parfumante selon l'une quelconque des revendications 5 à 7,
(b) fournir un ou plusieurs autre(s) composant(s) du produit parfumé à produire, et
(c) mettre en contact ou mélanger ledit (lesdits) autre(s) composant(s) fourni(s) à l'étape (b) avec une quantité sensoriellement efficace du (des) (i) composés ou du (ii) mélange ou de la (iii) composition de substance parfumante fournis à l'étape (a).

11. Utilisation
(i) du composé de formule (épi-I) tel que défini dans la revendication 1 et/ou du composé de formule (épi-II) tel que défini dans la revendication 1, ou
(ii) d'un mélange selon l'une quelconque des revendications 2 à 4, ou
(iii) d'une composition de substance parfumante selon l'une quelconque des revendications 5 à 7,
en tant que substance parfumante, de préférence pour conférer, modifier et/ou intensifier une ou plusieurs note(s) olfactive(s) choisie(s) dans le groupe constitué par les notes boisées, d'ambre gris, d'ambre blanc, florales, épicées, fruitées, musquées, animales, aromatiques, aldéhydiques et d'agrumes.

12. Utilisation
(i) du composé de formule (épi-I) tel que défini dans la revendication 1 et/ou du composé de formule (épi-II) tel que défini dans la revendication 1, ou
(ii) d'un mélange selon l'une quelconque des revendications 2 à 4, ou
(iii) d'une composition de substance parfumante selon l'une quelconque des revendications 5 à 7,
afin de
(a) masquer ou réduire la ou une ou plusieurs impression(s) olfactive(s) désagréable(s) d'une ou de plusieurs substance(s) à odeur désagréable, et/ou
(b) intensifier la ou une ou plusieurs impression(s) olfactive(s) agréable(s) d'une ou de plusieurs substance(s) à odeur agréable, de préférence pour intensifier une ou plusieurs note(s) olfactive(s) choisie(s) dans le groupe constitué par les notes boisées, d'ambre gris, d'ambre blanc, florales, épicées, fruitées, musquées, animales, aromatiques, aldéhydiques et d'agrumes.

13. Utilisation d'un produit parfumé selon la revendication 8 ou 9 pour parfumer les textiles, les cheveux, la peau, les surfaces et/ou l'air ambiant.

14. Procédé destiné à fournir et/ou à intensifier une ou plusieurs note(s) olfactive(s) choisie(s) dans le groupe constitué par l'ambre gris, l'ambre blanc et les notes boisées dans/pour un produit, de préférence un produit parfumé tel que défini dans la revendication 8 ou 9, comprenant les ou constitué des étapes suivantes:
(a) identifier un produit auquel/dans lequel une ou plusieurs note(s) olfactive(s) choisie(s) dans le groupe constitué par l'ambre gris, l'ambre blanc et les notes boisées doi(ven)t être fournie(s) et/ou améliorée(s), de préférence un produit parfumé tel que défini dans la revendication 8 ou 9, et
(b) incorporer
(i) le composé de formule (épi-I) tel que défini dans la revendication 1 et/ou le composé de formule (épi-II) tel que défini dans la revendication 1, ou
(ii) un mélange selon l'une quelconque des revendications 2 à 4, ou
(iii) une composition de substance parfumante selon l'une quelconque des revendications 5 à 7,
dans le produit en une quantité suffisante afin de fournir et/ou intensifier une ou plusieurs note(s) olfactive(s) choisie(s) dans le groupe constitué par l'ambre gris, l'ambre blanc et les notes boisées, pour/dans le produit.

15. Procédé
(a) destiné à intensifier la ou une ou plusieurs impression(s) olfactive(s) agréable(s) d'une ou de plusieurs substance(s) à odeur agréable,
et/ou
(b) destiné à masquer ou à réduire la ou une ou plusieurs impression(s) olfactive(s) désagréable(s) d'une ou de plusieurs substances à odeur désagréable,
comprenant l'étape suivante consistant à:
mélanger la/les (a) substance(s) à odeur agréable et/ou (b) à odeur désagréable avec
(i) le composé de formule (épi-I) tel que défini dans la revendication 1 et/ou le composé de formule (épi-II) tel que défini dans la revendication 1, ou
(ii) un mélange selon l'une quelconque des revendications 2 à 4, ou
(iii) une composition de substance parfumante selon l'une quelconque des revendications 5 à 7,
la quantité du (des) (i) composé(s) ou du (ii) mélange ou de la (iii) composition de substance parfumante étant suffisante pour (a) intensifier la/les impression(s) olfactive(s) agréable(s) de la/des substance(s) à odeur agréable et/ou pour (b) masquer ou réduire la/les impression(s) olfactive(s) désagréable(s) de la/des substance(s) à odeur désagréable.
